(19) Europäisches Patentamt
European Patent Office
Office européen des brevets

(11) Veröffentlichungsnummer: 0 362 634 A2

(12) EUROPÄISCHE PATENTANMELDUNG

(21) Anmeldenummer: 89117496.3

(22) Anmeldetag: 21.09.89

(51) Int. Cl.5: C07D 211/90 , C07D 401/12 , A61K 31/44

(30) Priorität: 05.10.88 DE 3833894

(43) Veröffentlichungstag der Anmeldung: 11.04.90 Patentblatt 90/15

(84) Benannte Vertragsstaaten: AT BE CH DE ES FR GB GR IT LI LU NL SE

(71) Anmelder: BAYER AG

D-5090 Leverkusen 1 Bayerwerk(DE)

(72) Erfinder: Schwenner, Eckhard, Dr.
Paul-Ehrlich-Strasse 29
D-5600 Wuppertal 1(DE)
Erfinder: Gross, Rainer, Prof. Dr.
Platzhoffstrasse 23
D-5600 Wuppertal 1(DE)
Erfinder: Hebisch, Siegbert, Dr.
Richard-Seel-Weg 11
D-5600 Wuppertal 1(DE)
Erfinder: Schramm, Matthias, Dr.
Paffrather Strasse 38
D-5000 Köln 80(DE)
Erfinder: Bechem, Martin, Dr.
Obere Bergerheide 4
D-5600 Wuppertal 1(DE)
Erfinder: Hirth, Claudia, Dr.
Claudiusweg 9
D-5600 Wuppertal 1(DE)
Erfinder: Stasch, Johannes-Peter, Dr.
Schneewittchenweg 37
D-5600 Wuppertal 1(DE)

(54) **Basische Dihydropyridine, Verfahren zu ihrer Herstellung und ihre Verwendung als Zwischenprodukte.**

(57) Die Erfindung betrifft neue basische Dihydropyridinderivate der allgemeinen Formel I

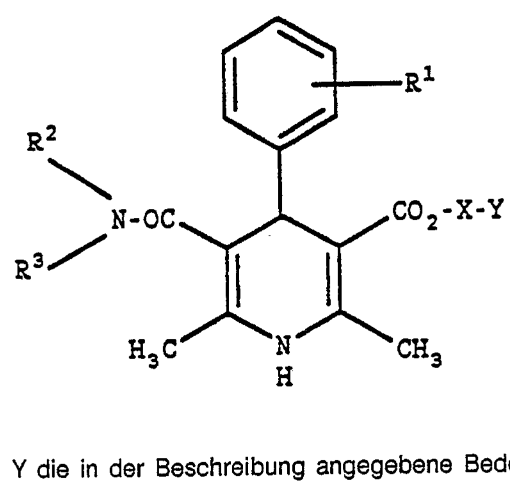

(I)

in welcher $R^1$ bis $R^3$, X und Y die in der Beschreibung angegebene Bedeutung haben, Verfahren zu ihrer Herstellung und ihre Verwendung zur Herstellung von teilweise bekannten 3-Carbonsäureaminoalkyl-dihydropyridinen.

EP 0 362 634 A2

## Basische Dihydropyridine Verfahren zu ihrer Herstellung und ihre Verwendung als Zwischenprodukte

Die Erfindung betrifft neue basische Dihydropyridinderivate, Verfahren zu ihrer Herstellung und ihre Verwendung zur Herstellung von teilweise bekannten 3-Carbonsäureaminoalkyl-dihydropyridinen.

Es ist bereits bekannt, daß die Darstellung von 1,4-Dihydropyridin-hydroxyalkylaminen über bestimmte Dihydropyridinamine als Zwischenstufen verläuft [vgl. US SN 66 49 04].

Die vorliegende Erfindung betrifft neue basische Dihydropyridine der allgemeinen Formel (I)

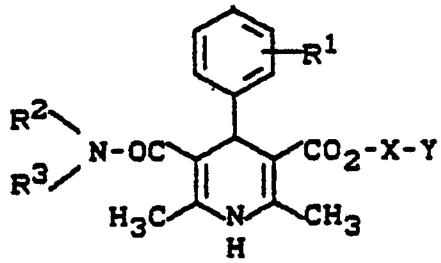

(I)

in welcher

$R^1$ - für Wasserstoff, Halogen, Alkyl mit bis zu 10 Kohlenstoffatomen, Alkoxy mit bis zu 10 Kohlenstoffatomen, Alkylthio mit bis zu 6 Kohlenstoffatomen, Cyano, Amino, Alkylamino mit bis zu 8 Kohlenstoffatomen, Dialkylamino mit jeweils bis zu 8 Kohlenstoffatomen je Alkylgruppe, Trifluormethyl, Trifluormethoxy, Difluormethoxy oder für Trifluormethylthio steht,

$R^2$ und $R^3$ gleich oder verschieden sind und jeweils

- für Wasserstoff,
- für Cycloalkyl mit 3 bis 8 Kohlenstoffatomen oder
- für geradkettiges oder verzweigtes Alkyl oder Alkenyl mit jeweils bis zu 18 Kohlenstoffatomen stehen, die gegebenenfalls substituiert sind durch Halogen, Hydroxy, Alkoxy mit bis zu 8 Kohlenstoffatomen, Alkylthio mit bis zu 8 Kohlenstoffatomen, Alkylcarbonyl mit bis zu 8 Kohlenstoffatomen im Alkylrest, Carboxy oder Alkoxycarbonyl mit bis zu 8 Kohlenstoffatomen, Cyano oder durch Phenyl- oder Phenoxygruppen, welche gegebenenfalls durch Nitro, Cyano, Trifluormethyl, Trifluormethoxy, Alkyl mit bis zu 4 Kohlenstoffatomen oder Alkoxy mit bis zu 4 Kohlenstoffatomen substituiert sind, oder wobei die Reste Cycloalkyl, Alkyl oder Alkenyl gegebenenfalls durch eine Gruppe der Formel $-NR^4R^5$ substituiert sind,

worin

$R^4$ und $R^5$ gleich oder verschieden sind, und jeweils Wasserstoff, Alkyl mit bis zu 8 Kohlenstoffatomen, Aralkyl mit 7 bis 14 Kohlenstoffatomen, Aryl mit 6 bis 12 Kohlenstoffatomen, Acetyl oder Benzoyl bedeuten,

oder $R^2$ und $R^3$ jeweils

- für Aryl mit 6 bis 10 Kohlenstoffatomen stehen, das bis zu 3-fach gleich oder verschieden durch Nitro, Cyano, Halogen, Alkyl mit bis zu 6 Kohlenstoffatomen, Alkoxy mit bis zu 6 Kohlenstoffatomen, Alkylthio mit bis zu 6 Kohlenstoffatomen, Trifluormethyl, Trifluormethoxy, Difluormethoxy, Trifluormethylthio, Amino, Alkylamino mit bis zu 4 Kohlenstoffatomen, Dialkylamino mit jeweils bis zu 4 Kohlenstoffatomen je Alkylgruppe, Acetylamino oder durch Benzoylamino substituiert ist, oder
- für einen 5- bis 7-gliedrigen gesättigten oder ungesättigten heterocyclischen Ring stehen, der als zusätzliches Heteroatom ein Sauerstoffatom, ein Schwefelatom oder eine NH- oder N-Alkyl-Gruppe (1-4 C-Atome) enthalten kann,

X - für einen geradkettigen, verzweigten oder cyclischen, gesättigten oder ungesättigten Kohlenwasserstoffrest mit bis zu 12 Kohlenstoffatomen steht, der gegebenenfalls durch ein Sauerstoff- oder Schwefelatom oder eine Gruppe $N-R^6$ unterbrochen ist, in der

$R^6$ - Wasserstoff, Alkyl bis zu 4 Kohlenstoffatomen, Benzyl oder Phenethyl bedeuten kann,

und/oder

der substituiert sein kann durch Halogen, Hydroxy, Acetoxy, Carboxy, Alkoxycarbonyl mit bis zu 8 Kohlenstoffatomen oder Phenyl, das gegebenenfalls substituiert ist durch Halogen, Alkyl mit bis zu 6 Kohlenstoffatomen, Alkoxy mit bis zu 6 Kohlenstoffatomen, Halogenmethyl, Halogenmethoxy, Hydroxy oder Cyano,

Y - für eine Gruppe der Formel

$-NR^7-Z$ oder für Phthalimido steht,

worin
$R^7$ - für Wasserstoff, $C_1$-$C_8$-Alkyl, $C_7$-$C_{14}$-Aralkyl oder für Phenyl steht, das gegebenenfalls durch Halogen, Nitro, Cyano oder $C_1$-$C_6$-Alkoxy substituiert ist und
Z - für eine Aminoschutzgruppe steht.

Aminoschutzgruppe steht im Rahmen der Erfindung für die üblich verwendeten Aminoschutzgruppen.

Hierzu gehören bevorzugt: Benzyloxycarbonyl, 4-Brombenzyloxycarbonyl, 2-Chlorbenzyloxycarbonyl, 3-Chlorbenzyloxycarbonyl, Dichlorbenzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, 3,4,5-Trimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert-Butoxycarbonyl, Pentoxycarbonyl, Isopentoxycarbonyl, Hexoxycarbonyl, Cyclohexoxycarbonyl, Octoxycarbonyl, 2-Ethylhexoxycarbonyl, 2-Iodhexoxycarbonyl, 2-Bromethoxycarbonyl, 2-Chlorethoxycarbonyl, 2,2,2-Trichlorethoxycarbonyl, 2,2,2-Trichlor-tert-butoxycarbonyl, Benzhydryloxycarbonyl, Bis-(4-methoxyphenyl)methoxycarbonyl, Phenacyloxycarbonyl, 2-Trimethylsilylethoxycarbonyl, 2-(Di-n-butyl-methylsilyl)-ethoxycarbonyl, 2-Triphenylsilylethoxycarbonyl, 2-(Dimethyl-tert-butylsilyl)ethoxycarbonyl, Menthyloxycarbonyl, Vinyloxycarbonyl, Allyloxycarbonyl, Phenoxycarbonyl, Tolyloxycarbonyl, 2,4-Dinitrophenoxycarbonyl, 4-Nitrophenoxycarbonyl, 2,4,5-Trichlorphenoxycarbonyl, Naphthyloxycarbonyl, Fluorenyl-9-methoxycarbonyl, Ethylthiocarbonyl, Methylthiocarbonyl, Butylthiocarbonyl, tert-Butylthiocarbonyl, Phenylthiocarbonyl, Benzylthiocarbonyl, Methylaminocarbonyl, Ethylaminocarbonyl, Propylaminocarbonyl, iso-Propylaminocarbonyl, Formyl, Acetyl, Propionyl, Pivaloyl, 2-Chloracetyl, 2-Bromacetyl, 2- Iodacetyl, 2,2,2-Trifluoracetyl, 2,2,2-Trichloracetyl, Benzoyl, 4-Chlorbenzoyl, 4-Methoxybenzoyl, 4-Nitrobenzyl, Naphthylcarbonyl, Phenoxyacetyl, Adamantylcarbonyl, Dicyclohexylphosphoryl, Diphenylphosphoryl, Dibenzylphosphoryl, Di-(4-nitrobenzyl)phosphoryl, Phenoxyphenylphosphoryl, Diethylphosphinyl, Diphenylphosphinyl, Phthaloyl oder Phthalimido.

Von besonderem Interesse sind die Aminoschutzgruppen Phthalimido, Butoxycarbonyl, tert.-Butoxycarbonyl.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise, z.B. durch Kristallisation, Chromatographie oder Craig-Verteilung in die stereoisomer einheitlichen Bestandteile trennen (vgl. E.L. Eliel, Stereochemistry of Carbon Compounds, McGraw Hill, 1962).

Bevorzugt sind Verbindungen der allgemeinen Formel (I),
in welcher
$R^1$ - für Wasserstoff, Fluor, Chlor, Brom, Alkyl mit bis zu 8 Kohlenstofatomen, Alkoxy mit bis zu 8 Kohlenstoffatomen, Alkylthio mit bis zu 4 Kohlenstoffatomen, Cyano, Amino, Alkylamino mit bis zu 6 Kohlenstoffatomen, Dialkylamino mit jeweils bis zu 6 Kohlenstoffatomen je Alkylgruppe, Trifluormethyl, Trifluormethoxy, Difluormethoxy oder für Trifluormethylthio steht,
$R^2$ und $R^3$ gleich oder verschieden sind und jeweils
- für Wasserstoff, Cycloalkyl mit 3 bis 7 Kohlenstoffatomen stehen oder
- für geradkettiges oder verzweigtes Alkyl oder Alkenyl mit bis zu 14 Kohlenstoffatomen stehen, die substituiert sein können durch Fluor, Chlor, Brom, Hydroxy, Alkoxy mit bis zu 6 Kohlenstoffatomen, Alkylthio mit bis zu 6 Kohlenstoffatomen, Alkylcarbonyl mit bis zu 6 Kohlenstoffatomen im Alkylrest, Carboxy, Alkoxycarbonyl mit bis zu 6 Kohlenstoffatomen, durch gegebenenfalls durch Nitro, Trifluormethyl, Methyl oder Methoxy substituierte Phenyl- oder Phenoxy-Rest, oder wobei die Reste Alkyl und Alkenyl gegebenenfalls substituiert sind durch Cyano und/oder durch eine Gruppe der Formel $-NR^4R^5$,
worin
$R^4$ und $R^5$ gleich oder verschieden sind und jeweils Wasserstoff, Alkyl mit bis zu 6 Kohlenstoffatomen, Benzyl, Phenethyl, Phenyl, Acetyl oder Benzoyl bedeuten,
oder $R^2$ und $R^3$ jeweils
- für Phenyl oder Naphthyl stehen, die bis zu 3-fach gleich oder verschieden substituiert sein können durch Nitro, Fluor, Chlor, Brom, Alkyl mit bis zu 4 Kohlenstoffatomen, Alkoxy mit bis zu 4 Kohlenstoffatomen, Alkylthio mit bis zu 4 Kohlenstoffatomen, Trifluormethyl, Trifluormethoxy, Amino, Alkylamino mit bis zu 6 Kohlenstoffatomen, Dialkylamino mit jeweils bis zu 6 Kohlenstoffatomen je Alkylgruppe, Acetylamino oder durch Benzoylamino, oder
- für Pyrrolidino, Pyridino, Morpholino oder für Piperazino, N-$C_1$-$C_4$-Alkylpiperazino, N-$C_7$-$C_9$-Aralkyl- oder N-Phenyl-piperazino stehen,
und

X - für einen geradkettigen, verzweigten oder cyclischen, gesättigten oder ungesättigten Kohlenwasserstoffrest mit bis zu 10 Kohlenstoffatomen steht, der gegebenenfalls durch ein Sauerstoff- oder Schwefelatom oder eine Gruppe N-$R^6$ unterbrochen ist, in der

$R^6$ - Wasserstoff, Alkyl bis zu 2 Kohlenstoffatomen, Benzyl oder Phenethyl bedeuten kann,

und/oder

der substituiert sein kann durch Halogen, Hydroxy, Acetoxy, Carboxy, Alkoxycarbonyl mit bis zu 4 Kohlenstoffatomen oder durch Phenyl, das gegebenenfalls substituiert ist durch Halogen, Alkyl mit bis zu 4 Kohlenstoffatomen, Alkoxy mit bis zu 4 Kohlenstoffatomen, Trifluormethyl oder Trifluormethoxy,

Y - für eine Gruppe der Formel

-$NR^7$-Z oder für Phthalimido steht,

worin

$R^7$ - für Wasserstoff, $C_1$-$C_6$-Alkyl, $C_7$-$C_{10}$-Aralkyl oder für Phenyl steht, das gegebenenfalls durch Halogen, Nitro, Cyano oder $C_1$-$C_4$-Alkoxy substituiert ist und

Z - für eine Aminoschutzgruppe aus der Gruppe Butyloxycarbonyl, tert.-Butyloxycarbonyl oder Phthalimido, steht.

Besonders bevorzugt sind Verbindungen der allgemeinen Formel (I),

in welcher

$R^1$ - für Wasserstoff, Fluor, Chlor, Alkyl mit bis zu 6 Kohlenstoffatomen, Alkoxy mit bis zu 6 Kohlenstoffatomen, Alkylthio mit bis zu 3 Kohlenstoffatomen, Cyano, Amino, Alkylamino mit bis zu 4 Kohlenstoffatomen, Dialkylamino mit jeweils bis zu 4 Kohlenstoffatomen je Alkylgruppe, Trifluormethyl, Trifluormethoxy, Difluormethoxy oder für Trifluormethylthio steht,

$R^2$ und $R^3$ gleich oder verschieden sind und jeweils

- für Wasserstoff, Cyclopropyl, Cyclopentyl oder Cyclohexyl stehen, oder

- für geradkettiges oder verzweigtes Alkyl oder Alkenyl mit bis zu 10 Kohlenstoffatomen stehen, das substituiert sein kann durch Fluor, Chlor, Hydroxy, Alkoxy mit bis zu 4 Kohlenstoffatomen, Alkylthio mit bis zu 4 Kohlenstoffatomen, Alkylcarbonyl mit bis zu 4 Kohlenstoffatomen im Alkylrest, Carboxy, Alkoxycarbonyl mit bis zu 4 Kohlenstoffatomen, Phenoxy, Phenyl oder die obengenannten Reste Alkyl und Alkenyl gegebenenfalls substituiert sind durch eine Gruppe der Formel -$NR^4R^5$,

worin

$R^4$ und $R^5$ gleich oder verschieden sind und Wasserstoff, Alkyl mit bis zu 4 Kohlenstoffatomen, Benzyl, Phenyl oder Acetyl bedeuten,

oder $R^2$ und $R^3$ jeweils

für Phenyl stehen, das bis zu 2-fach gleich oder verschieden substituiert sein kann durch Nitro, Fluor, Chlor, Methyl, Methoxy, Trifluormethyl, Trifluormethoxy, Amino, Alkylamino mit bis zu 2 Kohlenstoffatomen oder Dialkylamino mit jeweils bis zu 2 Kohlenstoffatomen je Alkylgruppe steht,

X - für einen geradkettigen, verzeigten oder cyclischen, gesättigten oder ungesättigten Kohlenwasserstoffrest mit bis zu 8 Kohlenstoffatomen steht, der gegebenenfalls durch ein Sauerstoff-oder Schwefelatom oder eine Gruppe N-$R^6$ unterbrochen ist, in der

$R^6$ - Wasserstoff, Alkyl mit bis zu 2 Kohlenstoffatomen, Benzyl oder Phenethyl bedeuten kann,

und/oder substituiert sein kann durch Hydroxy, Carboxy, Alkoxycarbonyl mit bis zu 4 Kohlenstoffatomen oder durch Phenyl, das gegebenenfalls substituiert sein kann durch Halogen, Methyl, Methoxy, Trifluormethyl oder Trifluormethoxy,

Y - für eine Gruppe der Formel

-$NR^7$-Z oder für Phthalimido steht,

worin

$R^7$ - für Wasserstoff, $C_1$-$C_4$-Alkyl, $C_7$-$C_9$-Aralkyl oder für Phenyl steht, das gegebenenfalls durch Fluor, Chlor, Nitro, Cyano, Methoxy, Ethoxy oder Propoxy substituiert ist und

Z - für tert.-Butyloxycarbonyl oder Phthalimido steht.

Die erfindungsgemäßen Verbindungen der allgemeinen Formel (I)

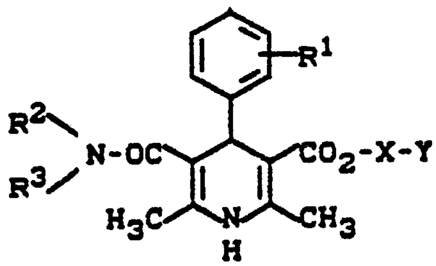

(I)

in welcher
$R^1$, $R^2$, $R^3$, X und Y die oben angegebene Bedeutung haben,
erhält man, indem man
Aldehyde der allgemeinen Formel (II)

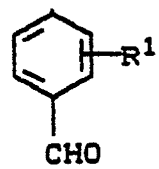

(II)

in welcher
$R^1$ die oben angegebene Bedeutung hat,
mit β-Ketocarbonsäureamiden der allgemeinen Formel (III)

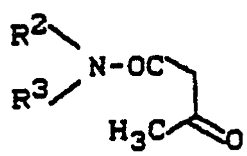

(III)

in welcher
$R^2$ und $R^3$ die oben angegebene Bedeutung haben,
gegebenenfalls nach Isolierung der hieraus entstehenden Ylidenverbindungen der allgemeinen Formel (IV)

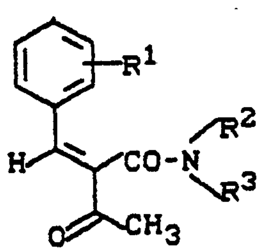

(IV)

in welcher
$R^1$, $R^2$ und $R^3$ die oben angegebene Bedeutung haben,
mit Aminocrotonsäureestern der allgemeinen Formel (V)

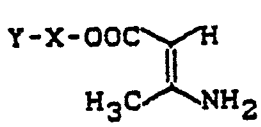

(V)

in welcher
X und Y die oben angegebene Bedeutung haben,

gegebenenfalls in Gegenwart von inerten organischen Lösemitteln bei Temperaturen zwischen 10 °C und 150 °C umsetzt.

Die Synthese für die erfindungsgemäßen Verbindungen kann durch folgendes Formelschema wiedergegeben werden:

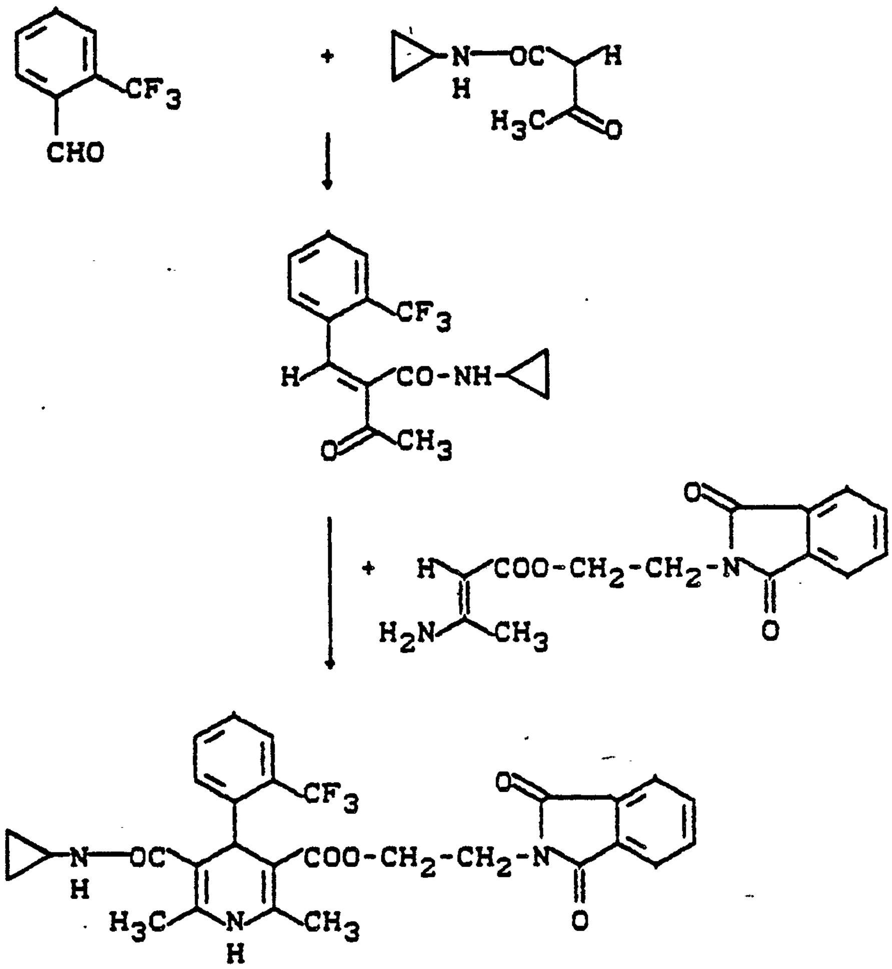

Als Lösemittel kommen Wasser oder alle inerten organischen Lösemittel in Frage, die sich unter den Reaktionsbedingungen nicht verändern. Hierzu gehören vorzugsweise Alkohole wie Methanol, Ethanol, Propanol, Isopropanol, Ether wie Diethylether, Dioxan, Tetrahydrofuran, Glykolmonomethylether oder Glykoldimethylether, oder Amide wie Dimethylformamid, Dimethylacetamid oder Hexamethylphosphorsäuretriamid, oder Eisessig, Dimethylsulfoxid, Acetonitril oder Pyridin.

Die Reaktionstemperaturen können in einem größeren Bereich variiert werden. Im allgemeinen arbeitet man zwischen +10 °C und +150 °C, vorzugsweise zwischen +20 °C und +100 °C, insbesondere bei der Siedetemperatur des jeweiligen Lösemittels.

Die Umsetzung kann bei Normaldruck, aber auch bei erhöhtem oder erniedrigtem Druck durchgeführt werden. Im allgemeinen arbeitet man bei Normaldruck.

Das Verhältnis der an der Reaktion beteiligten Stoffe ist beliebig. Im allgemeinen arbeitet man jedoch mit molaren Mengen der Reaktanden. Die Isolierung und Reinigung der erfindungsgemäßen Substanzen erfolgt vorzugsweise derart, daß man das Lösemittel im Vakuum abdestilliert und den gegebenenfalls erst nach Eiskühlung kristallin erhaltenen Rückstand aus einem geeigneten Lösemittel umkristallisiert. In einigen Fällen kann es erforderlich sein, die erfindungsgemäßen Verbindungen durch Chromatographie zu reinigen.

Die als Ausgangsstoffe eingesetzten Aldehyde der allgemeinen Formel (II) sind bekannt oder können nach bekannten Methoden hergestellt werden [DOS 21 65 260; 24 01 665; T.D. Harris, G.P. Roth, J. Org. Chem. 44, 2004 (1979); W.J. Dale, H.E. Hennis, J. Am. Chem. Soc. 78, 2543 (1956); Chem. Abstr. 59, 13929 (1963)].

Die Verbindungen der allgemeinen Formel (III) sind bekannt oder können nach bekannten Methden

hergestellt werden [DOS 11 42 859, EP 22 06 53].

Die Yliden-β-Ketocarbonsäurederivate der allgemeinen Formel (IV) sind bekannt oder können nach bekannten Methoden hergestellt werden [G. Jones "The Knoevenagel Condensation" in Organic Reactions, Bd. XV, 204 (1967)].

Die eingesetzten Enaminocarbonsäureester der allgemeinen Formel (V) sind bekannt oder können nach bekannten Methoden hergestellt werden [F.A. Glickman, A.C. Cope, J. Am. Chem. Soc., 67, 1017 (1945)].

Die erfindungsgemäßen Verbindungen der allgemeinen Formel (I) sind also Zwischenprodukte z.B. zur Darstellung von 3-Carbonsäure-aminoalkyl-dihydropyridinen der allgemeinen Formel (VI)

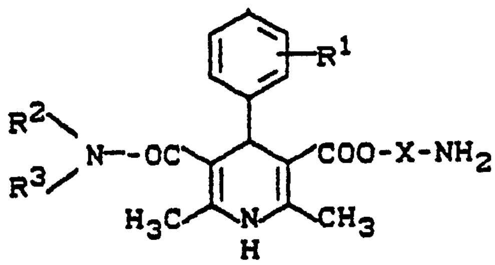 (VI)

in welcher

$R^1$, $R^2$, $R^3$ und X die oben angegebene Bedeutung haben, verwendbar.

Die Verbindungen der allgemeinen Formel (VI) erhält man, indem man Verbindungen der allgemeinen Formel (I) unter Abspaltung der Aminoschutzgruppe in an sich bekannter Weise, deblockiert, beispielsweise durch Hydrierung mit Hilfe von Palladium/Tierkohle, unter sauren Bedingungen, wenn Z für Benzyl steht, oder wenn Z für den Phthalimidorest steht, mit Hydrazinhydrat in organischen Lösemitteln wie Ethern, z.B. Tetrahydrofuran oder Dioxan, oder Alkoholen wie z.B. Methanol, Ethanol oder Isopropanol.

Die Verbindungen der allgemeinen Formel (VI) sind teilweise bekannt [vgl. EP 179 386].

Die Verbindungen der allgemeinen Formel (VI) und ihre physiologisch unbedenklichen Salze finden Verwendung bei der Bekämpfung der Hypertonie oder der Herzinsuffizienz.

## Ausführungsbeispiele

### A. Herstellung der neuen Zwischenprodukte

#### Beispiel 1

4-(3-Chlorphenyl)-1,4-dihydro-2,6-dimethyl-pyridin-3-carbonsäure-(2-phthalimidoethyl)-ester-5-carbonsäure-cyclopropylamid

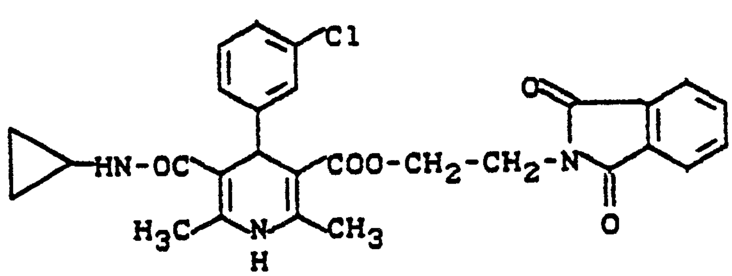

7,29 g (27,6 mmol) 3-Chlor-benzyliden-acetessigsäure-cyclopropylamid werden zusammen mit 7,56 g (27,6 mmol) Aminocrotonsäure-(2-phthalimidoethyl)-ester 24 Stunden unter Rückfluß gerührt. Nach Abkühlen und Einengen der Reaktionslösung wird das Produktgemisch säulenchromatographisch an Kieselgel (40

- 63 $\mu m$) mit dem Laufmittelgemisch Methylenchlorid/Methanol im Volumenverhältnis 30:1 gereinigt. Die nach Einengen des Eluats erhaltene amorphe Substanz wird im Vakuum getrocknet.

Ausbeute: 7,8 g (54,5% der Theorie)

$R_f$ : 0,51 (Methylenchlorid/Methanol = 10:1)

Analog Beispiel 1 werden die in Tabelle 1 aufgeführtenn Beispiele hergestellt:

**Tabelle 1**

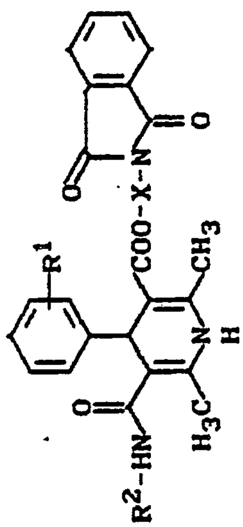

| Beispiel-Nr. | $R^1$ | $R^2$ | X | F [°C] | $R_f^*$ |
|---|---|---|---|---|---|
| 2 | o-$CF_3$ | 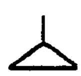 | $-CH_2-CH_2-$ | | 0,49 |
| 3 | o-$CF_3$ | $C_2H_5$ | $-CH_2-CH_2-$ | | 0,52 |
| 4 | m-Cl | $C_2H_5$ | $-CH_2-CH_2-$ | | 0,54 |
| 5 | m-Cl | $C_2H_5$ | $-CH-CH_2-$<br>$CH_3$ | 120-122 | 0,57 |
| 6 | m-Cl | 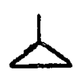 | $-CH-CH_2-$<br>$CH_3$ | | 0,51 |
| 7 | o-$CF_3$ | $C_2H_5$ | $-CH-CH_2-$<br>$CH_3$ | 114 | 0,44 |
| 8 | o-$CF_3$ | 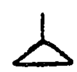 | $-CH-CH_2-$<br>$CH_3$ | 112 | 0,49 |

$R_f^*$ = HPTLC Fertigplatten; Kieselgel 60 $F_{254}$; Lösemittel: Methylenchlorid/Methanol 10:1

B. Verwendung der neuen Zwischenprodukte zur Herstellung von Dihydropyridin-Wirkstoffen

Beispiel 9

4-(3-Chlorphenyl)-1,4-dihydro-2,6-dimethyl-pyridin-3-carbonsäure-(2-aminoethyl)-ester-5-carbonsäure-cyclopropylamid

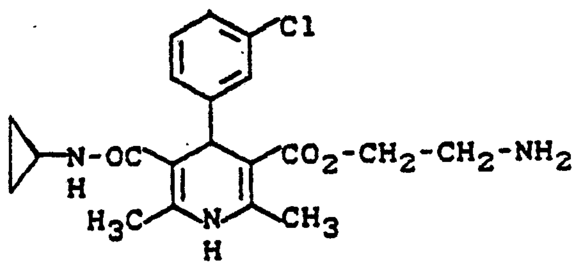

Eine Lösung von 6,6 g (12,7 mmol) 1,4-Dihydro-2,6-dimethyl-4-(3-chlorphenyl)-pyridin-3-carbonsäure-(2-phthalimidoethyl)-ester-5-carbonsäure-cyclopropylamid und 63,5 mmol Hydrazinhydrat wird 1 Stunde unter Rückfluß erhitzt. Nach Abkühlung wird der ausgefallene Niederschlag abfiltriert und mit Methylenchlorid gewaschen. Das Filtrat wird im Vakuum eingeengt und der Rückstand zunächst in Methylenchlorid aufgenommen, dann 1 mal mit 2 N Kaliumhydroxidlösung und 3 mal mit Wasser gewaschen. Die Lösung wird über Natriumsulfat getrocknet und im Vakuum eingeengt. Das Produkt kristallisiert beim Verreiben mit Ether.

Ausbeute: 3,6 g (72,7% der Theorie)

Schmp.: 168 - 170 °C

$R_f$ = 0,19 (Methylenchlorid/Methanol 5:1)

Die in Tabelle 2 aufgeführten Beispiele wurden analog Beispiel 9 hergestellt:

<u>**Tabelle 2**</u>

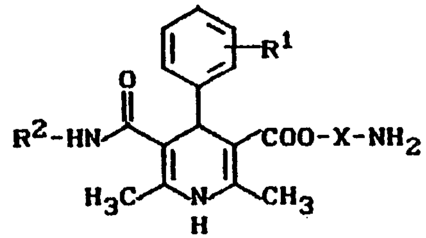

| Beispiel-Nr. | $R^1$ | $R^2$ | X | F [°C] | $R_f$* |
|---|---|---|---|---|---|
| 10 | m-Cl | $C_2H_5$ | $-CH_2-CH_2-$ | 136-139 | 0,20 |
| 11 | o-$CF_3$ | $C_2H_5$ | $-CH_2-CH_2-$ | 196-198 | 0,18 |
| 12 | o-$CF_3$ | 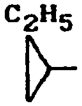 | $-CH_2-CH_2-$ | 218-220 | 0,17 |
| 13 | m-Cl | $C_2H_5$ | $-CH-CH_2-$<br>$CH_3$ | 154-164 | 0,57 |
| 14 | m-Cl | $C_2H_5$ | $-CH-CH_2-$<br>$CH_3$ | | 0,15 |
| 15 | m-Cl | 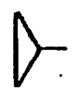 | $-CH-CH_2-$<br>$CH_3$ | 163-167 | 0,33 |
| 16 | m-Cl | 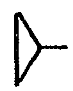 | $-CH-CH_2-$<br>$CH_3$ | | 0,15 |

$R_f$* = HPTLC Fertigplatten; Kieselgel 60 $F_{254}$; Lösemittel: Methylenchlorid/Methanol 10:1

**Fortsetzung Tabelle 2:**

| Beispiel-Nr. | $R^1$ | $R^2$ | X | F [°C] | $R_f$* |
|---|---|---|---|---|---|
| 17 | $o-CF_3$ | $C_2H_5$ | $-CH-CH_2-$<br>$CH_3$ | 93-95 | 0,32 |
| 18 | $o-CF_3$ | 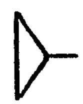 | $-CH-CH_2-$<br>$CH_3$ | 125-127 | 0,34 |
| 19 | $o-CF_3$ | 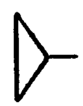 | $-CH-CH_2-$<br>$CH_3$ | | 0,16 |

$R_f$* = HPTLC Fertigplatten; Kieselgel 60 $F_{254}$; Lösemittel: Methylenchlorid/Methanol 5:1

## Ansprüche

1. Basische Dihydropyridine der allgemeinen Formel (I)

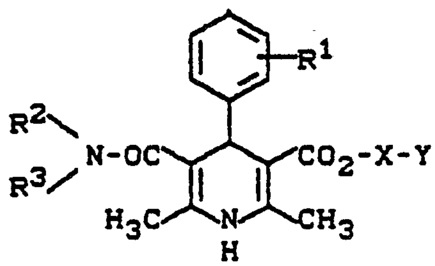

(I)

in welcher

$R^1$ - für Wasserstoff, Halogen, Alkyl mit bis zu 10 Kohlenstoffatomen, Alkoxy mit bis zu 10 Kohlenstoffatomen, Alkylthio mit bis zu 6 Kohlenstoffatomen, Cyano, Amino, Alkylamino mit bis zu 8 Kohlenstoffatomen, Dialkylamino mit jeweils bis zu 8 Kohlenstoffatomen je Alkylgruppe, Trifluormethyl, Trifluormethoxy, Difluormethoxy oder für Trifluormethylthio steht,

$R^2$ und $R^3$ gleich oder verschieden sind und jeweils

- für Wasserstoff,
- für Cycloalkyl mit 3 bis 8 Kohlenstoffatomen oder
- für geradkettiges oder verzweigtes Alkyl oder Alkenyl mit jeweils bis zu 18 Kohlenstoffatomen stehen, die gegebenenfalls substituiert sind durch Halogen, Hydroxy, Alkoxy mit bis zu 8 Kohlenstoffatomen, Alkylthio mit bis zu 8 Kohlenstoffatomen, Alkylcarbonyl mit bis zu 8 Kohlenstoffatomen im Alkylrest, Carboxy oder Alkoxycarbonyl mit bis zu 8 Kohlenstoffatomen, Cyano oder durch Phenyl- oder Phenoxygruppen, welche gegebenenfalls durch Nitro, Cyano, Trifluormethyl, Trifluormethoxy, Alkyl mit bis zu 4 Kohlenstoffatomen oder Alkoxy mit bis zu 4 Kohlenstoffatomen substituiert sind, oder wobei die Reste Cycloalkyl, Alkyl oder Alkenyl gegebenenfalls durch eine Gruppe der Formel $-NR^4R^5$ substituiert sind,

worin

$R^4$ und $R^5$ gleich oder verschieden sind, und jeweils Wasserstoff, Alkyl mit bis zu 8 Kohlenstoffatomen, Aralkyl mit 7 bis 14 Kohlenstoffatomen, Aryl mit 6 bis 12 Kohlenstoffatomen, Acetyl oder Benzoyl bedeuten,

oder $R^2$ und $R^3$ jeweils

- für Aryl mit 6 bis 10 Kohlenstoffatomen stehen,

das bis zu 3-fach gleich oder verschieden durch Nitro, Cyano, Halogen, Alkyl mit bis zu 6 Kohlenstoffatomen, Alkoxy mit bis zu 6 Kohlenstoffatomen, Alkylthio mit bis zu 6 Kohlenstoffatomen, Trifluormethyl, Trifluormethoxy, Difluormethoxy, Trifluormethylthio, Amino, Alkylamino mit bis zu 4 Kohlenstoffatomen, Dialkylamino mit jeweils bis zu 4 Kohlenstoffatomen je Alkylgruppe, Acetylamino oder durch Benzoylamino substituiert ist, oder

- für einen 5- bis 7-gliedrigen gesättigten oder ungesättigten heterocyclischen Ring stehen, der als zusätzliches Heteroatom ein Sauerstoffatom, ein Schwefelatom oder eine NH-oder N-Alkyl-Gruppe (1-4 C-Atome) enthalten kann,

X - für einen geradkettigen, verzweigten oder cyclischen, gesättigten oder ungesättigten Kohlenwasserstoffrest mit bis zu 12 Kohlenstoffatomen steht, der gegebenenfalls durch ein Sauerstoff- oder Schwefelatom oder eine Gruppe $N-R^6$ unterbrochen ist, in der

$R^6$ - Wasserstoff, Alkyl bis zu 4 Kohlenstoffatomen, Benzyl oder Phenethyl bedeuten kann,

und/oder

der substituiert sein kann durch Halogen, Hydroxy, Acetoxy, Carboxy, Alkoxycarbonyl mit bis zu 8 Kohlenstoffatomen oder Phenyl, das gegebenenfalls substituiert ist durch Halogen, Alkyl mit bis zu 6 Kohlenstoffatomen, Alkoxy mit bis zu 6 Kohlenstoffatomen, Halogenmethyl, Halogenmethoxy, Hydroxy oder Cyano,

Y - für eine Gruppe der Formel

$-NR^7-Z$ oder für Phthalimido steht,

worin

$R^7$ - für Wasserstoff, $C_1$-$C_8$-Alkyl, $C_7$-$C_{14}$-Aralkyl oder für Phenyl steht, das gegebenenfalls durch Halogen,

Nitro, Cyano oder $C_1$-$C_6$-Alkoxy substituiert ist und
Z - für eine Aminoschutzgruppe steht.

2. Verbindungen der allgemeinen Formel (I) gemäß Anspruch 1, in welcher
$R^1$, $R^2$, $R^3$, X und Y die in Anspruch 1 angegebene Bedeutung haben, und
Z für eine der folgenden Aminoschutzgruppen steht:
Benzyloxycarbonyl, 4-Brombenzyloxycarbonyl, 2-Chlorbenzyloxycarbonyl, 3-Chlorbenzyloxycarbonyl, Dichlorbenzyloxycarbonyl,3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzylcarbonyl, 2-Nitro-4,5-dimethoxybenzyloxycarbonyl, 3,4,5-Trimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert-Butoxycarbonyl, Pentoxycarbonyl, Isopentoxycarbonyl, Hexoxycarbonyl, Cyclohexoxycarbonyl, Octoxycarbonyl, 2-Ethylhexoxycarbonyl, 2-Iodhexoxycarbonyl, 2-Bromethoxycarbonyl, 2-Chlorethoxycarbonyl, 2,2,2-Trichlorethoxycarbonyl, 2,2,2-Trichlor-tert-butoxycarbonyl, Benzhydryloxycarbonyl, Bis-(4-methoxyphenyl)methoxycarbonyl, Phenacyloxycarbonyl, 2-Trimethylsilylethoxycarbonyl, 2-(Di-n-butyl-methylsilyl)ethoxycarbonyl, 2-Triphenylsilylethoxycarbonyl, 2-(Dimethyl-tert-butylsilyl)ethoxycarbonyl, Menthyloxycarbonyl, Vinyloxycarbonyl, Allyloxycarbonyl, Phenoxycarbonyl, Tolyloxycarbonyl, 2,4-Dinitrophenoxycarbonyl, 4-Nitrophenoxycarbonyl, 2,4,5-Trichlorphenoxycarbonyl, Naphthyloxycarbonyl, Fluorenyl-9-methoxycarbonyl, Ethylthiocarbonyl, Methylthiocarbonyl, Butylthiocarbonyl, tert-Butylthiocarbonyl, Phenylthiocarbonyl, Benzylthiocarbonyl, Methylaminocarbonyl, Ethylaminocarbonyl, Propylaminocarbonyl, iso-Propylaminocarbonyl, Formyl, Acetyl, Propionyl, Pivaloyl, 2-Chloracetyl, 2-Bromacetyl, 2-Iodacetyl, 2,2,2-Trifluoracetyl, 2,2,2-Trichloracetyl, Benzoyl, 4-Chlorbenzoyl, 4-Methoxybenzoyl, 4-Nitrobenzyl, Naphthylcarbonyl, Phenoxyacetyl, Adamantylcarbonyl, Dicyclohexylphosphoryl, Diphenylphosphoryl, Dibenzylphosphoryl, Di-(4-nitrobenzyl)phosphoryl, Phenoxyphenylphosphoryl, Diethylphosphinyl, Diphenylphosphinyl, Phthaloyl oder Phthalimido.

3. Verbindungen der allgemeinen Formel (I) gemäß Anspruch 1, in welcher
$R^1$ - für Wasserstoff, Fluor, Chlor, Brom, Alkyl mit bis zu 8 Kohlenstofatomen, Alkoxy mit bis zu 8 Kohlenstoffatomen, Alkylthio mit bis zu 4 Kohlenstoffatomen, Cyano, Amino, Alkylamino mit bis zu 6 Kohlenstoffatomen, Dialkylamino mit jeweils bis zu 6 Kohlenstoffatomen je Alkylgruppe, Trifluormethyl, Trifluormethoxy, Difluormethoxy oder für Trifluormethylthio steht,
$R^2$ und $R^3$ gleich oder verschieden sind und jeweils
- für Wasserstoff, Cycloalkyl mit 3 bis 7 Kohlenstoffatomen stehen oder
- für geradkettiges oder verzweigtes Alkyl oder Alkenyl mit bis zu 14 Kohlenstoffatomen stehen, die substituiert sein können durch Fluor, Chlor, Brom, Hydroxy, Alkoxy mit bis zu 6 Kohlenstoffatomen, Alkylthio mit bis zu 6 Kohlen stoffatomen, Alkylcarbonyl mit bis zu 6 Kohlenstoffatomen im Alkylrest, Carboxy, Alkoxycarbonyl mit bis zu 6 Kohlenstoffatomen, durch gegebenenfalls durch Nitro, Trifluormethyl, Methyl oder Methoxy substituierte Phenyl- oder Phenoxy-Rest, oder wobei die Reste Alkyl und Alkenyl gegebenenfalls substituiert sind durch Cyano und/oder durch eine Gruppe der Formel $-NR^4R^5$,
worin
$R^4$ und $R^5$ gleich oder verschieden sind und jeweils Wasserstoff, Alkyl mit bis zu 6 Kohlenstoffatomen, Benzyl, Phenethyl, Phenyl, Acetyl oder Benzoyl bedeuten,
oder $R^2$ und $R^3$ jeweils
- für Phenyl oder Naphthyl stehen, die bis zu 3-fach gleich oder verschieden substituiert sein können durch Nitro, Fluor, Chlor, Brom, Alkyl mit bis zu 4 Kohlenstoffatomen, Alkoxy mit bis zu 4 Kohlenstoffatomen, Alkylthio mit bis zu 4 Kohlenstoffatomen, Trifluormethyl, Trifluormethoxy, Amino, Alkylamino mit bis zu 6 Kohlenstoffatomen, Dialkylamino mit jeweils bis zu 6 Kohlenstoffatomen je Alkylgruppe, Acetylamino oder durch Benzoylamino, oder
- für Pyrrolidino, Pyridino, Morpholino oder für Piperazino, N-$C_1$-$C_4$-Alkylpiperazino, N-$C_7$-$C_9$-Aralkyl- oder N-Phenyl-piperazino stehen,
und
X - für einen geradkettigen, verzweigten oder cyclischen, gesättigten oder ungesättigten Kohlenwasserstoffrest mit bis zu 10 Kohlenstoffatomen steht,
der gegebenenfalls durch ein Sauerstoff- oder Schwefelatom oder eine Gruppe $N$-$R^6$ unterbrochen ist, in der
$R^6$ - Wasserstoff, Alkyl bis zu 2 Kohlenstoffatomen, Benzyl oder Phenethyl bedeuten kann,
und/oder
der substituiert sein kann durch Halogen, Hydroxy, Acetoxy, Carboxy, Alkoxycarbonyl mit bis zu 4 Kohlenstoffatomen oder durch Phenyl, das gegebenenfalls substituiert ist durch Halogen, Alkyl mit bis zu 4 Kohlenstoffatomen, Alkoxy mit bis zu 4 Kohlenstoffatomen, Trifluormethyl oder Trifluormethoxy,
Y - für eine Gruppe der Formel

$-NR^7-Z$ oder für Phthalimido steht,
worin
$R^7$ - für Wasserstoff, $C_1$-$C_6$-Alkyl, $C_7$-$C_{10}$-Aralkyl oder für Phenyl steht, das gegebenenfalls durch Halogen, Nitro, Cyano oder $C_1$-$C_4$-Alkoxy substituiert ist und
Z - für eine Aminoschutzgruppe aus der Gruppe tert.-Butyloxycarbonyl, Phthalimido oder Butyloxycarbonyl steht.

4. Verbindungen der allgemeinen Formel (I) gemäß Anspruch 1, in welcher
$R^1$ - für Wasserstoff, Fluor, Chlor, Alkyl mit bis zu 6 Kohlenstoffatomen, Alkoxy mit bis zu 6 Kohlenstoffatomen, Alkylthio mit bis zu 3 Kohlenstoffatomen, Cyano, Amino, Alkylamino mit bis zu 4 Kohlenstoffatomen, Dialkylamino mit jeweils bis zu 4 Kohlenstoffatomen je Alkylgruppe, Trifluormethyl, Trifluormethoxy, Difluormethoxy oder für Trifluormethylthio steht,
$R^2$ und $R^3$ gleich oder verschieden sind und jeweils
- für Wasserstoff, Cyclopropyl, Cyclopentyl oder Cyclohexyl stehen, oder
- für geradkettiges oder verzweigtes Alkyl oder Alkenyl mit bis zu 10 Kohlenstoffatomen stehen, das substituiert sein kann durch Fluor, Chlor, Hydroxy, Alkoxy mit bis zu 4 Kohlenstoffatomen, Alkylthio mit bis zu 4 Kohlenstoffatomen, Alkylcarbonyl mit bis zu 4 Kohlenstoffatomen im Alkylrest, Carboxy, Alkoxycarbonyl mit bis zu 4 Kohlenstoffatomen, Phenoxy, Phenyl oder die obengenannten Reste Alkyl und Alkenyl gegebenenfalls substituiert sind durch eine Gruppe der Formel $-NR^4R^5$,
worin
$R^4$ und $R^5$ gleich oder verschieden sind und Wasserstoff, Alkyl mit bis zu 4 Kohlenstoffatomen, Benzyl, Phenyl oder Acetyl bedeuten,
oder $R^2$ und $R^3$ jeweils
für Phenyl stehen, das bis zu 2-fach gleich oder verschieden substituiert sein kann durch Nitro, Fluor, Chlor, Methyl, Methoxy, Trifluormethyl, Trifluormethoxy, Amino, Alkylamino mit bis zu 2 Kohlenstoffatomen oder Dialkylamino mit jeweils bis zu 2 Kohlenstoffatomen je Alkylgruppe steht,
X - für einen geradkettigen, verzeigten oder cyclischen, gesättigten oder ungesättigten Kohlenwasserstoffrest mit bis zu 8 Kohlenstoffatomen steht, der gegebenenfalls durch ein Sauerstoff-oder Schwefelatom oder eine Gruppe $N-R^6$ unterbrochen ist, in der
$R^6$ - Wasserstoff, Alkyl mit bis zu 2 Kohlenstoffatomen, Benzyl oder Phenethyl bedeuten kann,
und/oder substituiert sein kann durch Hydroxy, Carboxy, Alkoxycarbonyl mit bis zu 4 Kohlenstoffatomen oder durch Phenyl, das gegebenenfalls substituiert sein kann durch Halogen, Methyl, Methoxy, Trifluormethyl oder Trifluormethoxy,
Y - für eine Gruppe der Formel
$-NR^7-Z$ oder für Phthalimido steht,
worin
$R^7$- für Wasserstoff, $C_1$-$C_4$-Alkyl, $C_7$-$C_9$-Aralkyl oder für Phenyl steht, das gegebenenfalls durch Fluor, Chlor, Nitro, Cyano, Methoxy, Ethoxy oder Propoxy substituiert ist und
Z - für tert.-Butyloxycarbonyl oder Phthalimido steht.

5. Verfahren zur Herstellung von basischen Dihydropyridinen der allgemeinen Formel (I)

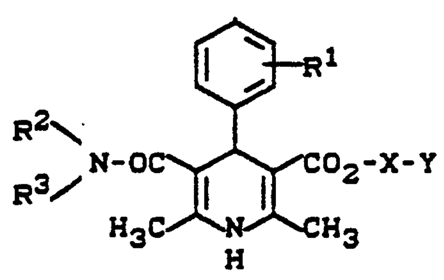

(I)

in welcher
$R^1$ - für Wasserstoff, Halogen, Alkyl mit bis zu 10 Kohlenstoffatomen, Alkoxy mit bis zu 10 Kohlenstoffatomen, Alkylthio mit bis zu 6 Kohlenstoffatomen, Cyano, Amino, Alkylamino mit bis zu 8 Kohlenstoffatomen, Dialkylamino mit jeweils bis zu 8 Kohlenstoffatomen je Alkylgruppe, Trifluormethyl, Trifluormethoxy, Difluormethoxy oder für Trifluormethylthio steht,
$R^2$ und $R^3$ gleich oder verschieden sind und jeweils
- für Wasserstoff,
- für Cycloalkyl mit 3 bis 8 Kohlenstoffatomen oder

- für geradkettiges oder verzweigtes Alkyl oder Alkenyl mit jeweils bis zu 18 Kohlenstoffatomen stehen, die gegebenenfalls substituiert sind durch Halogen, Hydroxy, Alkoxy mit bis zu 8 Kohlenstoffatomen, Alkylthio mit bis zu 8 Kohlenstoffatomen, Alkylcarbonyl mit bis zu 8 Kohlenstoffatomen im Alkylrest, Carboxy oder Alkoxycarbonyl mit bis zu 8 Kohlenstoffatomen, Cyano oder durch Phenyl- oder Phenoxygruppen, welche gegebenenfalls durch Nitro, Cyano, Trifluormethyl, Trifluormethoxy, Alkyl mit bis zu 4 Kohlenstoffatomen oder Alkoxy mit bis zu 4 Kohlenstoffatomen substituiert sind, oder wobei die Reste Cycloalkyl, Alkyl oder Alkenyl gegebenenfalls durch eine Gruppe der Formel $-NR^4R^5$ substituiert sind,
worin
$R^4$ und $R^5$ gleich oder verschieden sind, und jeweils Wasserstoff, Alkyl mit bis zu 8 Kohlenstoffatomen, Aralkyl mit 7 bis 14 Kohlenstoffatomen, Aryl mit 6 bis 12 Kohlenstoffatomen, Acetyl oder Benzoyl bedeuten,
oder $R^2$ und $R^3$ jeweils
- für Aryl mit 6 bis 10 Kohlenstoffatomen stehen,
das bis zu 3-fach gleich oder verschieden durch Nitro, Cyano, Halogen, Alkyl mit bis zu 6 Kohlenstoffatomen, Alkoxy mit bis zu 6 Kohlenstoffatomen, Alkylthio mit bis zu 6 Kohlenstoffatomen, Trifluormethyl, Trifluormethoxy, Difluormethoxy, Trifluormethylthio, Amino, Alkylamino mit bis zu 4 Kohlenstoffatomen, Dialkylamino mit jeweils bis zu 4 Kohlenstoffatomen je Alkylgurppe, Acetylamino oder durch Benzoylamino substituiert ist, oder
- für einen 5- bis 7-gliedrigen gesättigten oder ungesättigten heterocyclischen Ring stehen, der als zusätzliches Heteroatom ein Sauerstoffatom, ein Schwefelatom oder eine NH-oder N-Alkyl-Gruppe (1-4 C-Atome) enthalten kann,
X - für einen geradkettigen, verzweigten oder cyclischen, gesättigten oder ungesättigten Kohlenwasserstoffrest mit bis zu 12 Kohlenstoffatomen steht, der gegebenenfalls durch ein Sauerstoff- oder Schwefelatom oder eine Gruppe $N-R^6$ unterbrochen ist, in der
$R^6$ - Wasserstoff, Alkyl bis zu 4 Kohlenstoffatomen, Benzyl oder Phenethyl bedeuten kann,
und/oder
der substituiert sein kann durch Halogen, Hydroxy, Acetoxy, Carboxy, Alkoxycarbonyl mit bis zu 8 Kohlenstoffatomen oder Phenyl, das gegebenenfalls substituiert ist durch Halogen, Alkyl mit bis zu 6 Kohlenstoffatomen, Alkoxy mit bis zu 6 Kohlenstoffatomen, Halogenmethyl, Halogenmethoxy, Hydroxy oder Cyano,
Y - für eine Gruppe der Formel
$-NR^7-Z$ oder für Phthalimido steht,
worin
$R^7$ - für Wasserstoff, $C_1$-$C_8$-Alkyl, $C_7$-$C_{14}$-Aralkyl oder für Phenyl steht, das gegebenenfalls durch Halogen, Nitro, Cyano oder $C_1$-$C_6$-Alkoxy substituiert ist und
Z - für eine Aminoschutzgruppe steht,
dadurch gekennzeichnet, daß man
Aldehyde der allgemeinen Formel (II)

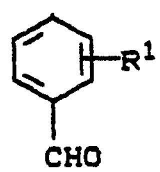

(II)

in welcher
$R^1$ die oben angegebene Bedeutung hat,
mit $\beta$-Ketocarbonsäureamiden der allgemeinen Formel (III)

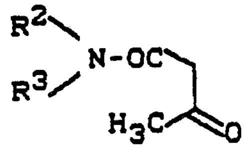

(III)

in welcher
$R^2$ und $R^3$ die oben angegebene Bedeutung haben,

gegebenenfalls nach Isolierung der hieraus entstehenden Ylidenverbindungen der allgemeinen Formel (IV)

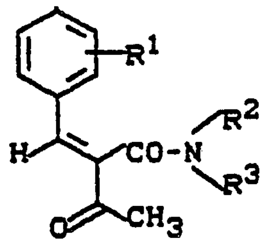

(IV)

in welcher
$R^1$, $R^2$ und $R^3$ die oben angegebene Bedeutung haben,
mit Aminocrotonsäureestern der allgemeinen Formel (V)

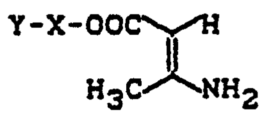

(V)

in welcher
X und Y die oben angegebene Bedeutung haben,
gegebenenfalls in Gegenwart von inerten organischen Lösemitteln bei Temperaturen zwischen 10° C und 150° C umsetzt.

6. Verwendung von Verbindungen der allgemeinen Formel (I) gemäß Anspruch 1 als Zwischenprodukte bei der Herstellung von 3-Carbonsäure-aminoalkyl-dihydropyridinen der allgemeinen Formel (VI)

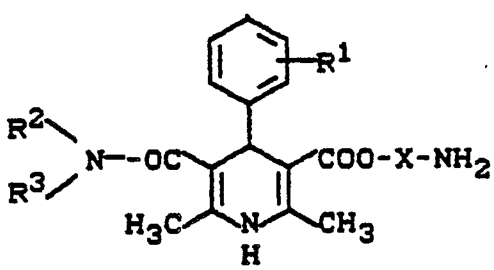

(VI)

in welcher
$R^1$, $R^2$, $R^3$ und X die im Anspruch 1 angegebene Bedeutung haben.

7. Verfahren zur Herstellung von Verbindungen der allgemeinen Formel (VI)

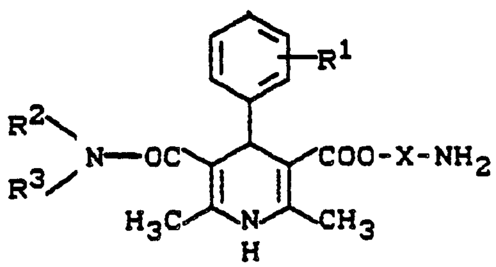

(VI)

in welcher
$R^1$, $R^2$, $R^3$ und X und im Anspruch 5 angegebene Bedeutung haben,
dadurch gekennzeichnet, daß man basische Dihydropyridine der allgemeinen Formel (I)

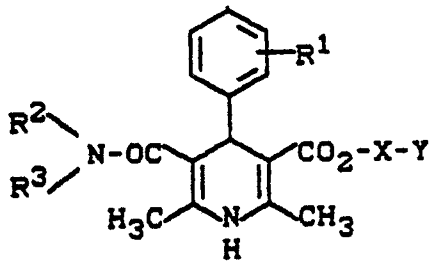

(I)

in welcher
$R^1$, $R^2$, $R^3$ und X die im Anspruch 5 angegebene Bedeutung haben und
Y - für eine Gruppe der Formel
$-NR^7-Z$ oder für Phthalimido steht,
worin
$R^7$ - für Wasserstoff, $C_1$-$C_8$-Alkyl, $C_7$-$C_{14}$-Aralkyl oder für Phenyl steht, das gegebenenfalls durch Halogen, Nitro, Cyano oder $C_1$-$C_6$-Alkoxy substituiert ist und
Z - für eine Aminoschutzgruppe steht,
unter Abspaltung der Aminoschutzgruppe deblockiert.

8. Verfahren zur Herstellung von Verbindungen der allgemeinen Formel (VI) gemäß Anspruch 7, dadurch gekennzeichnet, daß man die Abspaltung der Aminoschutzgruppe durch Hydrierung mit Hilfe von Palladium-Tierkohle unter sauren Bedingungen durchführt, wenn Z für Benzyl steht
oder für den Fall, daß Z für den Phthalimidorest steht, die Abspaltung mit Hydrazinhydrat in organischen Lösemitteln wie Ethern oder Alkoholen durchführt.